# EUROPEAN PATENT APPLICATION

(11) **EP 2 087 888 A1**
(43) Date of publication of application: **12.08.2009**
(21) Application number: 09160652.5
(22) Date of filing: 24.02.2004
(51) Int. Cl.: A61K 31/138, A61K 31/522, A61K 31/135, A61K 31/40, A61K 31/47, A61K 31/403, A61K 31/55, A61K 31/675, A61K 31/472, A61K 31/4178, A61K 31/415, A61K 31/4184, A61K 31/41, A61P 9/04, A61P 9/10, A61P 13/12

(54) **Adenosine a1 receptor antagonists for the treatment of alkalosis**

(30) Priority: 24.02.2003 US 449953 P; 25.02.2003 US 450500 P; 25.02.2003 US 450499 P; 28.02.2003 US 451326 P; 21.04.2003 US 464813 P; 21.04.2003 US 464811 P; 21.04.2003 US 464812 P; 21.04.2003 US 464815 P
(62) Divisional of application: 04714224.5
(71) Applicant: Novacardia, Inc., San Diego, CA 92130 (US)
(72) Inventor: Otsuki, Lauren, San Diego, CA 92127 (US); Dittrich, Howard, San Diego, CA 92131 (US); Widder, Kenneth, J., Rancho Santa Fe, CA 92067 (US); Blantz, Roland, Del Mar, CA 92014 (US); Thomson, Scott, Poway, CA 92064 (US)
(74) Representative: Hussain, Deeba

(57) **Abstract**

The present invention relates to adenosine A₁ receptor antagonists (AA₁RA), for use in treating alkalosis.

## Description

### Related Applications

The present application claims priority to U.S. Provisional Application Serial No. 60/449,953, filed on February 24, 2003, by Dittrich et al., and entitled "METHOD OF TREATMENT OF DISEASE USING AN ANGIOTENSIN CONVERTING ENZYME INHIBITOR OR AN ANGIOTENSIN II RECEPTOR BLOCKER AND AN ADENOSINE A1 RECEPTOR ANTAGONIST AND A BETA-BLOCKER;" U.S. Provisional Application Serial No. 60/450,499, filed on February 25, 2003, by Dittrich et al., and entitled "METHOD OF TREATMENT OF DISEASE USING AN ADENOSINE A1 RECEPTOR ANTAGONIST AND A BETA-BLOCKER;" U.S. Provisional Application Serial No. 60/450,500, filed on February 25, 2003, by Dittrich et al., and entitled "USE OF ADENOSINE A1 RECEPTOR ANTAGONISTS IN PRE-DIABETIC PATIENTS AND EARLY DIABETES MELLITUS FOR THE PREVENTION OF DIABETIC NEPHORPATHY;" U.S. Provisional Application Serial No. 60/451,326, filed on February 28, 2003, by Dittrich et al., and entitled "METHOD OF TREATING ALKALOSIS USING ADENOSINE A1 RECEPTOR ANTAGONISTS;" U.S. Provisional Application Serial No. 60/464,811, filed on April 21, 2003, by Dittrich et al., and entitled "METHOD OF TREATING ALKALOSIS USING ADENOSINE A1 RECEPTOR ANTAGONISTS;" U.S. Provisional Application Serial No. 60/464,812, filed on April 21, 2003, by Dittrich et al., and entitled "METHOD OF TREATMENT OF DISEASE USING AN ANGIOTENSIN CONVERTING ENZYME INHIBITOR OR AN ANGIOTENSIN II RECEPTOR BLOCKER AND AN ADENOSINE A1 RECEPTOR ANTAGONIST AND A BETA-BLOCKER;" U.S. Provisional Application Serial No. 60/464,813, filed on April 21, 2003, by Dittrich et al., and entitled "USE OF ADENOSINE A1 RECEPTOR ANTAGONISTS IN PRE-DIABETIC PATIENTS AND EARLY DIABETES MELLITUS FOR THE PREVENTION OF DIABETIC NEPHORPATHY;" and U.S. Provisional Application Serial No. 60/464,815, filed on April 21, 2003, by Dittrich et al., and entitled "METHOD OF TREATMENT OF DISEASE USING AN ADENOSINE A1 RECEPTOR ANTAGONIST AND A BETA-BLOCKER;" all of which are incorporated by reference herein in their entirety.

### Field of the Invention

The present invention relates to pharmaceutical compositions comprising an adenosine A1 receptor antagonist, either alone, or in combination with other pharmaceuticals, and methods of treatment of patients suffering from cardiac disease and/or renal failure with said compositions.

### Summary of the Invention

Disclosed is a pharmaceutical composition comprising a beta-blocker and an adenosine A₁ receptor antagonist (AA₁RA).

In addition, disclosed is a pharmaceutical composition comprising an angiotensin converting enzyme (ACE) inhibitor and an adenosine A₁ receptor antagonist (AA₁RA).

Further disclosed is a pharmaceutical composition comprising an angiotensin II receptor blocker (ARB) and an adenosine A₁ receptor antagonist (AA₁RA).

Also disclosed is a method of treating cardiovascular disease or renal disease comprising identifying a patient in need of such treatment, and administering a pharmaceutical composition disclosed herein to said patient.

Disclosed herein are methods of treating alkalosis comprising identifying a patient in need thereof and administering an adenosine A₁ receptor antagonist (AA₁RA) to said patient. Also disclosed are pharmaceutical compositions comprising an AA₁RA.

Disclosed herein are methods of treating diabetic nephropathy comprising identifying a patient in need thereof and administering an adenosine A₁ receptor antagonist (AA₁RA) to said patient. Also disclosed are pharmaceutical compositions comprising an AA₁RA.

### Detailed Description of the Preferred Embodiment

AA₁RAs

Aspects of the present disclosure relate to methods of treating various diseases using an AA₁RA, either alone or in combination with one or more other pharmaceuticals. A number of AA₁RAs are known in the art, though currently, none are commercially available as a therapeutic. AA₁RAs antagonize the A₁ receptor of adenosine selectively. The majority of the known AA₁RAs are derivatives of xanthine and include compounds such as 1,3-dipropyl-8-{3-oxatricyclo[3.1.2.0.^{2,4}]oct-6(7)-yl}xanthine (also known as 1,3-dipropyl-8-[5,6-exo-epoxy-2(S) norbornyl]xanthine, ENX, CVT-124, and BG9719), 8-(3-noradamantyl)-1,3-dipropylxanthine (also known as KW-3902), theophylline, and caffeine. Other AA₁RAs are disclosed in U.S. Patent Nos. 5,446,046, 5,631,260, and 5,668,139, the specification of all of which is hereby incorporated by reference herein in their entirety, including any drawings. The scope of the present invention includes all those AA₁RAs now known and all those AA₁RAs to be discovered in the future.

The AA₁RA used in the pharmaceutical compositions or methods disclosed herein may be a xanthine-derivative compound. The xanthine-derivative compound may be a compound of Formula I or a pharmaceutically acceptable salt thereof, 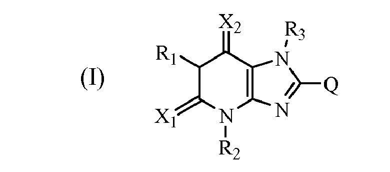 where
each of X₁ and X₂ independently represents oxygen or sulfur;
Q represents: 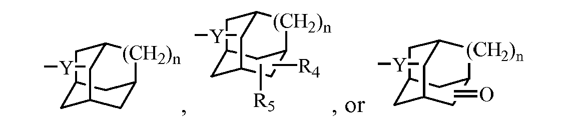 where Y represents a single bond or alkylene having 1 to 4 carbon atoms, n represents 0 or 1;
each of R₁ and R₂ independently represents hydrogen, lower alkyl, allyl, propargyl, or hydroxy-substituted, oxo-substituted or unsubstituted lower alkyl, and R₃ represents hydrogen or lower alkyl, or
R₄ and R₅ are the same or different and each represent hydrogen or hydroxy, and when both R₄ and R₅ are hydrogen, at least one of R₁ and R₂ is hydroxy-substituted or oxo-substituted lower alkyl, provided that when Q is 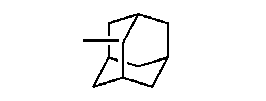 then R₁, R₂ and R₃ are not simultaneously methyl.

In some embodiments, both of R₁ and R₂ of the compound of Formula I are lower alkyl and R₃ is hydrogen; and both of X₁ and X₂ are oxygen. In other embodiments, R₁, R₂ and R₃ independently represents hydrogen or lower alkyl. In still other embodiments, each of R₁ and R₂ independently represents allyl or propargyl and R₃ represents hydrogen or lower alkyl. In certain embodiments, X₁ and X₂ are both oxygen and n is 0.

In some embodiments, R₁ is hydroxy-substituted, oxo-substituted or unsubstituted propyl; R₂ is hydroxy-substituted or unsubstituted propyl; and Y is a single bond. In other embodiments, R₁ is propyl, 2-hydroxypropyl, 2-oxopropyl or 3-oxopropyl; R₂ is propyl, 2-hydroxypropyl or 3-hydroxypropyl.

In some embodiments Q is 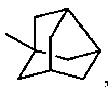 while in other embodiments Q is 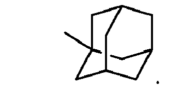 In other embodiments, Q is 9-hydroxy, 9-oxo or 6-hydroxy substituted 3tricyclo[3.3.1.0^{3,7}]nonyl, or 3-hydroxy-1tricyclo[3.3.1.1^{3,7}]decyl.

In certain embodiments, the AA₁RA is selected from the group consisting of 8-(noradamantan-3-yl)-1,3-dipropylxanthine; 1,3-Diallyl-8-(3-noradamantyl)xanthine, 3-allyl-8-(3-noradamantyl)-1-propargylxanthine, 8-(trans-9-hydroxy-3-tricyclo[3.3.1.0^{3,7}]nonyl) -1,3-dipropylxanthine, 8-(cis-9-hydroxy-3-tricyclo[3.3.1.0^{3,7}]nonyl)-1,3-dipropylxanthine, 8-(trans-9-hydroxy-3-tricyclo[3.3.1.0^{3,7}]nonyl)-1-(2-oxopropyl)-3-propylxanthine and 1-(2-hydroxypropyl)-8-(trans-9-hydroxy-3-tricyclo[3.3.1.0^{3,7}]nonyl)-3-propylxanthine, or a pharmaceutically acceptable salt thereof.

In other embodiments, the AA₁RA is a xanthine epoxide-derivative compound of Formula II or Formula III, or a pharmaceutically acceptable salt thereof, 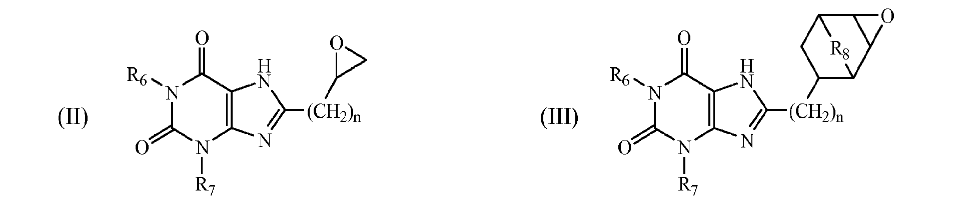 where R₆ and R₇ are the same or different, and can be hydrogen or an alkyl group of 1-4 carbons, R₈ is either oxygen or (CH₂)₁₋₄, and n=0-4.

The xanthine epoxide-derivative compound may be

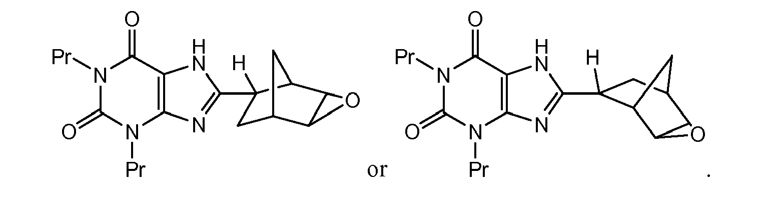

### Combinations with Beta-Blockers

Aspects of the present invention relate to the treatment of cardiovascular diseases using a combination of a beta-blocker and an adenosine A₁ receptor antagonists, or AA₁RA. Each of these compounds have individually been shown to be somewhat effective in the treatment of cardiovascular disease, such as congestive heart failure, hypertension, asymptomatic left ventricular dysfunction, or coronary artery disease.

A number of beta-blockers are commercially available. These compounds include, but are not limited to, acebutolol hydrochloride, atenolol, betaxolol hydrochloride, bisoprolol fumarate, carteolol hydrochloride, esmolol hydrochloride, metoprolol, metoprolol tartrate, nadolol, penbutolol sulfate, pindolol, propranolol hydrochloride, succinate, and timolol maleate. Beta-blockers, generally, are beta₁ and/or beta₂ adrenergic receptor blocking agents, which decrease the positive chronotropic, positive inotropic, bronchodilator, and vasodilator responses caused by beta-adrenergic receptor agonists. The scope of the present invention includes all those beta-blockers now known and all those beta-blockers to be discovered in the future.

A significant problem encountered in treating certain conditions with individual medications is that following a course of therapy the patients become refractory to the treatment, i.e., the patients begin to respond less and less to the medication until they do not respond at all. This problem is very common in patients who suffer from, for example, congestive heart failure, and are treated with diuretics.

Individual diuretics act on a specific segment of nephrons, e.g., proximal tubule, loop of Henle, or distal tubule. One mechanism by which diuretics increase urine volume is that they inhibit reabsorption of sodium and accompanying water passing through the nephron. Thus, for example, a loop diuretic inhibits reabsorption in the loop of Henle. As a consequence, higher concentrations of sodium are passed downstream to the distal tubule. This initially results in a greater volume of urine, hence the diuretic effect. However, the distal portion of the tubule recognizes the increase in sodium concentration and the kidney reacts in two ways; one is to increase sodium reabsorption elsewhere in the nephron; the other is to feedback via adenosine A₁ receptors to the afferent arteriole where vasoconstriction occurs. This feedback mechanism is known as tubuloglomerular feedback (TGF). This vasoconstriction results in decreased renal blood flow and decreased glomerular filtration rate (GFR). With time, these two mechanisms result in a decrease in diuretic effect and worsening of renal function. This sequence of events contributes to the progression of disease.

The present inventors have discovered that the combination of AA₁RAs and beta blockers is beneficial in either congestive heart failure (CHF) or hypertension, or any of the other indications set forth herein. Beta-blockers are known to have antihypertensive effects. While the exact mechanism of their action is unknown, possible mechanisms, such as reduction in cardiac output, reduction in plasma renin activity, and a central nervous system sympatholytic action, have been put forward. From various clinical studies, it is clear that administration of beta-blockers to patients with hypertension results initially in a decrease in cardiac output, little immediate change in blood pressure, and an increase in calculated peripheral resistance. With continued administration, blood pressure decreases within a few days, cardiac output remains reduced, and peripheral resistance falls toward pretreatment levels. Plasma renin activity is also reduced markedly in patients with hypertension, which will have an inhibitory action on the renin-angiotensin system, thus decreasing the after-load and allowing for more efficient forward function of the heart. The use of these compounds has been shown to increase survival rates among patients suffering from CHF or hypertension. The compounds are now part of the standard of care for CHF and hypertension.

AA₁RAs act on the afferent arteriole of the kidney to produce vasodilation and thereby improve renal blood flow in patients with CHF. They also block the TGF mechanism mediated by adenosine (via A₁ receptors) described above. This ultimately allows for increased GFR and improved renal function. In addition, AA₁RAs inhibit the reabsorption of sodium (and, therefore, water) in the proximal tubule, which results in diuresis.

The combination of the invention described herein acts synergistically to further improve the condition of patients with hypertension or CHF. The diuretic effect of AA₁RAs, specially in salt-sensitive hypertensive patients along with the blockage of beta adrenergic receptors decreases blood pressure through two different mechanisms, whose effects build on one another. In addition, most CHF patients are also on additional diuretics. The combination allows for greater efficacy of other more distally acting diuretics by improving renal blood flow and renal function.

Beta-blockers are well established in the treatment of hypertension. The addition of AA₁RAs will further treat hypertension via its diuretic effect from inhibiting sodium reabsorption through the proximal tubule. In addition, since many hypertensive patients are sodium sensitive, the addition of an AA₁RA to a beta-blocker will result in further blood pressure reduction. AA₁RA action on tubuloglomerular feedback further improves renal function to result in greater diuresis and lower blood pressure.

Thus, in one aspect, the present disclosure relates to a pharmaceutical composition comprising a beta-blocker and an adenosine A₁ receptor antagonist (AA₁RA). The beta-blocker may be selected from the group consisting of acebutolol hydrochloride, atenolol, betaxolol hydrochloride, bisoprolol fumarate, carteolol hydrochloride, esmolol hydrochloride, metoprolol, metoprolol tartrate, nadolol, penbutolol sulfate, pindolol, propranolol hydrochloride, succinate, and timolol maleate, or a pharmaceutically acceptable salt, prodrug, ester, or amide thereof. However, the inclusion of other beta-blockers is within the scope of the present invention.

In another aspect, the present disclosure relates to a method of treating cardiovascular disease or renal disease comprising identifying a patient in need of such treatment, and administering a pharmaceutical composition as described herein to said patient. In certain embodiments, the patient may be a mammal. The mammal may be selected from the group consisting of mice, rats, rabbits, guinea pigs, dogs, cats, sheep, goats, cows, primates, such as monkeys, chimpanzees, and apes, and humans. In some embodiments, the patient is a human.

In some embodiments, the administering step comprises administering said beta-blocker and said AA₁RA nearly simultaneously. These embodiments include those in which the AA₁RA and the beta-blocker are in the same administrable composition, i.e., a single tablet, pill, or capsule, or a single solution for intravenous injection, or a single drinkable solution, or a single dragee formulation or patch, contains both compounds. The embodiments also include those in which each compound is in a separate administrable composition, but the patient is directed to take the separate compositions nearly simultaneously, i.e., one pill is taken right after the other or that one injection of one compound is made right after the injection of another compound, etc.

In other embodiments the administering step comprises administering one of the beta-blocker and the AA₁RA first and then administering the other one of the beta-blocker and the AA₁RA. In these embodiments, the patient may be administered a composition comprising one of the compounds and then at some time, a few minutes or a few hours, later be administered another composition comprising the other one of the compounds. Also included in these embodiments are those in which the patient is administered a composition comprising one of the compounds on a routine or continuous basis while receiving a composition comprising the other compound occasionally.

The methods of the present invention are intended to provide treatment for cardiovascular disease, which may include congestive heart failure, hypertension, asymptomatic left ventricular dysfunction, atrial fibrillation, arrhythmia refractory to other modality, recurrent ventricular tachycardia, recurrent ventricular fibrillation, coronary artery disease, or acute myocardial infarction. In some instances, patients suffering from a cardiovascular disease are in need of after-load reduction. The methods of the present invention are suitable to provide treatment for these patients as well.

In another aspect, the invention relates to a pharmaceutical composition comprising a combination of an AA₁RA and a beta-blocker, as described above, and a physiologically acceptable carrier, diluent, or excipient, or a combination thereof.

### Combinations with Angiotensin Converting Enzyme Inhibitors or Angiotensin II Receptor Blockers

Aspects of the present invention relate to the treatment of renal and/or cardiac diseases using a combination of an adenosine A₁ receptor antagonists, or AA₁RA, and an angiotensin converting enzyme (ACE) inhibitor or an angiotensin II receptor blocker (ARB). Each of these compounds have individually been shown to be somewhat effective in the treatment of cardiac disease, such as congestive heart failure, hypertension, asymptomatic left ventricular dysfunction, or acute myocardial infarction, or renal disease, such as diabetic nephropathy, contrast-mediated nephropathy, toxin-induced renal injury, or oxygen free-radical mediated nephropathy.

A number of ACE inhibitors are commercially available. These compounds, whose chemical structure is somewhat similar, include lisinopril, enalapril, quinapril, ramipril, benazepril, captopril, fosinopril, moexipril, trandolapril, and perindopril. ACE inhibitors, generally, are compounds that inhibit the action of angiotensin converting enzyme, which converts angiotensin I to angiotensin II. The scope of the present invention includes all those ACE inhibitors now known and all those ACE inhibitors to be discovered in the future.

A number of ARBs are also commercially available or known in the art. These compounds include losartan, irbesartan, candesartan, telmisartan, eposartan, and valsartan. ARBs reduce blood pressure by relaxing blood vessels. This allows better blood flow. ARBs function stems from their ability to block the binding of angiotensin II, which would normally cause vessels to constrict. The scope of the present invention includes all those ARBs now known and all those ARBs to be discovered in the future.

The present inventors have discovered that the combination of AA₁RAs and ACE inhibitors or ARBs is beneficial in either congestive heart failure (CHF) or hypertension. The use of ACE inhibitors and ARBs in CHF relies on inhibition of renin-angiotensin system. These compounds decrease the after-load, thereby allowing for more efficient forward function of the heart. In addition, renal function is "normalized" or improved such that patients remove excess fluid more effectively. The use of these compounds has been shown to increase survival rates among patients suffering from CHF or hypertension. The compounds are now part of the standard of care for CHF and hypertension.

The combination of the invention described herein acts synergistically to further improve renal function for continued diuresis. In addition, most CHF patients are also on additional diuretics. The combination allows for greater efficacy of other more distally acting diuretics by improving renal blood flow and renal function.

Both ACE inhibitors and ARBs are well established in the treatment of hypertension via their action through the renin-angiotensin system. The addition of AA₁RAs will further treat hypertension via its diuretic effect from inhibiting sodium reabsorption through the proximal tubule. In addition, since many hypertensive patients are sodium sensitive, the addition of an AA₁RA to an ACE inhibitor or an ARB will result in further blood pressure reduction. AA₁RA action on tubuloglomerular feedback further improves renal function to result in greater diuresis and lower blood pressure.

ACE inhibitors and ARBs are also known to prevent some of the renal damage induced by the immunosuppresant, cyclosporin A. However, there is a renal damaging effect despite their use. The present inventors have discovered that the combination ACE inhibitors and ARBs with AA₁RAs would be more effective in preventing drug-induced nephrotoxicity, such as that induced by cyclosporin A, contrast medium (iodinated), and aminoglycoside antibiotics. In this setting there is renal vasoconstriction that can be minimized by both compounds. In addition, direct negative effects on the tubular epithelium by cyclosporin is less prominent in the setting of adenosine A₁ receptor antagonism, in that blocking A₁ receptors decreases active processes. Furthermore, there are fewer oxidative byproducts that are injurious to the tubular epithelium. In addition, the inhibitory effect of AA₁RA blockade on the tubuloglomerular feedback mechanism helps preserve function in the setting of nephrotoxic drugs.

It is known that ACE inhibitors and ARBs are beneficial in preventing the worsening of renal dysfunction in diabetics as measured by albuminuria (proteinuria). Once diabetes begins, glucosuria develops and the kidneys begin to actively reabsorb glucose, especially through the proximal convoluted tubule. This active process may result in oxidative stress and begin the disease process of diabetic nephropathy. Early manifestations of this process are hypertrophy and hyperplasia of the kidney. Ultimately, the kidney begins to manifest other signs such as microalbuminuria and decreased function. It is postulated that the active reabsorption of glucose is mediated in part by adenosine A₁ receptors. Blockade of this process by an AA₁RA limits or prevents the early damage manifested in diabetics.

The combination of AA₁RA and ACE inhibitors or ARBs, as disclosed herein, works to limit both early and subsequent damage to the kidneys in diabetes. The presently disclosed combinations are given at the time of diagnosis of diabetes or as soon as glycosuria is detected in at risk patients (metabolic syndrome). The long-term treatment using the combinations of the present invention includes daily administration of the pharmaceutical compositions described herein.

Thus, in a first aspect, the invention relates to a pharmaceutical composition comprising an angiotensin converting enzyme (ACE) inhibitor and an adenosine A₁ receptor antagonist (AA₁RA). The ACE inhibitor may be selected from the group consisting of lisinopril, enalapril, quinapril, ramipril, benazepril, captopril, fosinopril, moexipril, trandolapril, and perindopril, or a pharmaceutically acceptable salt, prodrug, ester, or amide thereof. However, the inclusion of other ACE inhibitors is within the scope of the present invention.

In another aspect, the invention relates to a pharmaceutical composition comprising an angiotensin II receptor blocker (ARB) and an adenosine A₁ receptor antagonist (AA₁RA). The ARB may be selected from the group consisting of losartan, irbesartan, candesartan, telmisartan, eposartan, and valsartan, or a pharmaceutically acceptable salt, prodrug, ester, or amide thereof. However, the inclusion of other ARBs is within the scope of the present invention.

In a further aspect, the invention relates to a pharmaceutical composition comprising an ACE inhibitor, an ARB, and an adenosine A₁ receptor antagonist (AA₁RA).

In another aspect, the invention relates to a method of treating cardiovascular disease or renal disease comprising identifying a patient in need of such treatment, and administering a pharmaceutical composition as described herein to said patient. In certain embodiments, the patient may be a mammal. The mammal may be selected from the group consisting of mice, rats, rabbits, guinea pigs, dogs, cats, sheep, goats, cows, primates, such as monkeys, chimpanzees, and apes, and humans. In some embodiments, the patient is a human.

In some embodiments, the administering step comprises administering said ACE inhibitor or said ARB and said AA₁RA nearly simultaneously. These embodiments include those in which the AA₁RA and the ACE inhibitor or ARB are in the same administrable composition, i.e., a single tablet, pill, or capsule, or a single solution for intravenous injection, or a single drinkable solution, or a single dragee formulation or patch, contains both compounds. The embodiments also include those in which each compound is in a separate administrable composition, but the patient is directed to take the separate compositions nearly simultaneously, i.e., one pill is taken right after the other or that one injection of one compound is made right after the injection of another compound, etc.

In other embodiments the administering step comprises administering one of the ACE inhibitor or ARB and the AA₁RA first and then administering the other one of the ACE inhibitor or ARB and the AA₁RA. In these embodiments, the patient may be administered a composition comprising one of the compounds and then at some time, a few minutes or a few hours, later be administered another composition comprising the other one of the compounds. Also included in these embodiments are those in which the patient is administered a composition comprising one of the compounds on a routine or continuous basis while receiving a composition comprising the other compound occasionally.

The methods of the present invention are intended to provide treatment for cardiovascular disease, which may include congestive heart failure, hypertension, asymptomatic left ventricular dysfunction, or acute myocardial infarction. In some instances, patients suffering from a cardiovascular disease are in need of after-load reduction. The methods of the present invention are suitable to provide treatment for these patients as well.

The methods of the present invention are also intended to provide treatment for renal disease, which may include renal hypertrophy, renal hyperplasia, microproteinuria, proteinuria, diabetic nephropathy, contrast-mediated nephropathy, toxin-induced renal injury, or oxygen free-radical mediated nephropathyhypertensive nephropathy, diabetic nephropathy, contrast-mediated nephropathy, toxin-induced renal injury, or oxygen free-radical mediated nephropathy.

In another aspect, the invention relates to a pharmaceutical composition comprising a combination of an AA₁RA and an ACE inhibitor or ARB, as described above, and a physiologically acceptable carrier, diluent, or excipient, or a combination thereof.

### Treatment of Alkalosis

Alkalosis is an acid-base disturbance caused by an elevation in plasma bicarbonate (HCO₃⁻) concentration. It is a primary pathophysiologic event characterized by the gain of bicarbonate or the loss of nonvolatile acid from extracellular fluid. The kidney preserves normal acid-base balance by two mechanisms: bicarbonate reclamation, mainly in the proximal tubule, and bicarbonate generation, predominantly in the distal nephron. Bicarbonate reclamation is mediated mainly by a Na⁺-H⁺ antiporter and to a smaller extent by the H⁺-ATPase (adenosine triphosphatase). The principal factors affecting HCO₃⁻ reabsorption include effective arterial blood volume, glomerular filtration rate, potassium, and partial pressure of carbon dioxide. Bicarbonate regeneration is primarily affected by distal Na⁺ delivery and reabsorption, aldosterone, systemic pH, ammonium excretion, and excretion of titratable acid.

There are a number of different types of alkalosis, for instance metabolic alkalosis and respiratory alkalosis. Respiratory alkalosis is a condition that affects mountain climbers in high altitude situations.

To generate metabolic alkalosis, either a gain of base or a loss of acid must occur. The loss of acid may be via the upper gastrointestinal tract or via the kidney. Excess base may be gained by oral or parenteral HCO₃⁻ administration or by lactate, acetate, or citrate administration.

Factors that help maintain metabolic alkalosis include decreased glomerular filtration rate, volume contraction, hypokalemia, and aldosterone excess. Clinical states associated with metabolic alkalosis are vomiting, mineralocorticoid excess, the adrenogenital syndrome, licorice ingestion, diuretic administration, and Bartter's and Gitelman's syndromes.

The two types of metabolic alkalosis (i.e., chloride-responsive, chloride-resistant) are classified based upon the amount of chloride in the urine. Chloride-responsive metabolic alkalosis involves urine chloride levels less than 10 mEq/L, and it is characterized by decreased extracellular fluid (ECF) volume and low serum chloride such as occurs with vomiting. This type responds to administration of chloride salt. Chloride-resistant metabolic alkalosis involves urine chloride levels more than 20 mEq/L, and it is characterized by increased ECF volume. As the name implies, this type resists administration of chloride salt. Ingestion of excessive oral alkali (usually milk plus calcium carbonate) and alkalosis complicating primary hyperaldosteronism are examples of chloride resistant alkalosis.

Many patients with edematous states are treated with diuretics. Unfortunately, with continued therapy, the patient's bicarbonate level increases and progressive alkalosis may ensue. Diuretics cause metabolic alkalosis by several mechanisms, including (1) acute contraction of the extracellular fluid (ECF) volume (NaCl excretion without HCO₃⁻), thereby increasing the concentration of HCO₃⁻ in the ECF; (2) diuretic-induced potassium and chloride depletion; and (3) secondary aldosteronism. Continued use of the diuretic or either of the latter two factors will maintain the alkalosis.

The addition of an AA₁RA allows continued diuresis and maintained renal function without worsening the alkalosis. The AA₁RA inhibits the active resorption of HCO₃⁻ across the proximal tubule of the kidney.

Thus, in one aspect, the present invention relates to a method of treating metabolic alkalosis comprising identifying a patient in need thereof and administering an adenosine A₁ receptor antagonist (AA₁RA) to said patient. In certain embodiments, the patient is suffering from high altitude mountain sickness. In some embodiments, the patient has edema. In some of these embodiments, the patient may be on diuretic therapy. The diuretic may be a loop diuretic, proximal diuretic, or distal diuretic. In other embodiments, the patient suffers from acid loss through the patient's upper gastrointestinal tract, for example, through excessive vomiting. In still other embodiments the patient has ingested excessive oral alkali. The methods of the present invention can be practiced with any compound that antagonizes adenosine A₁ receptors.

In another aspect, the invention relates to a pharmaceutical composition comprising a combination of an AA₁RA, as described above, and a physiologically acceptable carrier, diluent, or excipient, or a combination thereof.

### Treatment of Diabetic Nephropathy

Uncontrolled diabetes causes damage to many tissues of the body. Kidney damage caused by diabetes most often involves thickening and hardening (sclerosis) of the internal kidney structures, particularly the glomerulus (kidney membrane). Kimmelstiel-Wilson disease is the unique microscopic characteristic of diabetic nephropathy in which sclerosis of the glomeruli is accompanied by nodular deposits of hyaline.

The glomeruli are the site where blood is filtered and urine is formed. They act as a selective membrane, allowing some substances to be excreted in the urine and other substances to remain in the body. As diabetic nephropathy progresses, increasing numbers of glomeruli are destroyed, resulting in impaired kidney functioning. Filtration slows and protein, namely albumin, which is normally retained in the body, may leak in the urine. Albumin may appear in the urine for 5 to 10 years before other symptoms develop. Hypertension often accompanies diabetic nephropathy.

Diabetic nephropathy may eventually lead to the nephrotic syndrome (a group of symptoms characterized by excessive loss of protein in the urine) and chronic renal failure. The disorder continues to progress, with end-stage renal disease developing, usually within 2 to 6 years after the appearance of renal insufficiency with proteinuria.

The mechanism that causes diabetic nephropathy is unknown. It may be caused by inappropriate incorporation of glucose molecules into the structures of the basement membrane and the tissues of the glomerulus. Hyperfiltration (increased urine production) associated with high blood sugar levels may be an additional mechanism of disease development.

The diabetic nephropathy is the most common cause of chronic renal failure and end stage renal disease in the United States. About 40% of people with insulin-dependent diabetes will eventually develop end-stage renal disease. 80% of people with diabetic nephropathy as a result of insulin-dependent diabetes mellitus (IDDM) have had this diabetes for 18 or more years. At least 20% of people with non-insulin-dependent diabetes mellitus (NIDDM) will develop diabetic nephropathy, but the time course of development of the disorder is much more variable than in IDDM. The risk is related to the control of the blood-glucose levels. Risk is higher if glucose is poorly controlled than if the glucose level is well controlled.

Diabetic nephropathy is generally accompanied by other diabetic complications including hypertension, retinopathy, and vascular (blood vessel) changes, although these may not be obvious during the early stages of nephropathy. Nephropathy may be present for many years before nephrotic syndrome or chronic renal failure develops. Nephropathy is often diagnosed when routine urinalysis shows protein in the urine.

Current treatments for diabetic nephropathy include administration of angiotensin converting enzyme inhibitors (ACE Inhibitors) during the more advanced stages of the disease. Currently there is no treatment in the earlier stages of the disease since ACE inhibitors may not be effective when the disease is symptom-free (i.e., when the patient only shows proteinuria).

Although the mechanism implicated in early renal disease in diabetics is that of hyperglycemia, a potential mechanism may be related to the active reabsorption of glucose in the proximal tubule. This reabsorption is dependent in part on adenosine A₁ receptors.

AA₁RAs act on the afferent arteriole of the kidney to produce vasodilation and thereby improve renal blood flow in patients with diabetes. This ultimately allows for increased GFR and improved renal function. In addition, AA₁RAs inhibit the reabsorption of glucose in the proximal tubule in patients with newly diagnosed diabetic mellitus or in patients at risk for the condition (metabolic syndrome).

Thus, in one aspect, the present invention relates to a method of treating diabetic nephropathy comprising identifying a patient in need thereof and administering an adenosine A₁ receptor antagonist (AA₁RA) to said patient. In certain embodiments the patient is pre-diabetic, whereas in other embodiments the patient is in early stage diabetes. In some embodiments the patient suffers from insulin-dependent diabetes mellitus (IDDM), whereas in other embodiments the patient suffers from non-insulin-dependent diabetes mellitus (NIDDM).

In certain embodiments, the methods of the present invention are used to prevent or reverse renal hypertrophy. In other embodiments, the methods of the present invention are used to prevent or reverse renal hyperplasia. In still other embodiments, the methods of the present invention are used to ameliorate microproteinuria or proteinuria.

Before people develop type II diabetes, i.e., NIDDM, they almost always have "pre-diabetes." Pre-diabetic patients have blood glucose levels that are higher than normal but not yet high enough to be diagnosed as diabetes. For instance, the blood glucose level of pre-diabetic patients is between 110-126 mg/dL, using the fasting plasma glucose test (FPG), or between 140-200 mg/dL using the oral glucose tolerance test (OGTT). Blood glucose levels below 110 or 140, using FPG or OGTT, respectively, is considered normal, whereas individuals with blood glucose levels higher than 126 or 200, using FPG or OGTT, respectively, are considered diabetic. The methods of the present invention can be practiced with any compound that antagonizes adenosine A₁ receptors.

In certain aspects, the methods of the present invention can be practiced using a combination therapy, i.e., where the AA1RA compound of the invention is administered to the patient in combination with a second compound. In certain embodiments the second compound may be selected from a protein kinase C inhibitor, an inhibitor of tissue proliferation, an antioxidant, an inhibitor of glycosylation, and an endothelin B receptor inhibitor.

### Pharmaceutical Compositions

The term "pharmaceutical composition" refers to a mixture of a compound of the invention with other chemical components, such as diluents or carriers. The pharmaceutical composition facilitates administration of the compound to an organism. Multiple techniques of administering a compound exist in the art including, but not limited to, oral, injection, aerosol, parenteral, and topical administration. Pharmaceutical compositions can also be obtained by reacting compounds with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid and the like.

The term "carrier" defines a chemical compound that facilitates the incorporation of a compound into cells or tissues. For example dimethyl sulfoxide (DMSO) is a commonly utilized carrier as it facilitates the uptake of many organic compounds into the cells or tissues of an organism.

The term "diluent" defines chemical compounds diluted in water that will dissolve the compound of interest as well as stabilize the biologically active form of the compound. Salts dissolved in buffered solutions are utilized as diluents in the art. One commonly used buffered solution is phosphate buffered saline because it mimics the salt conditions of human blood. Since buffer salts can control the pH of a solution at low concentrations, a buffered diluent rarely modifies the biological activity of a compound.

The term "physiologically acceptable" defines a carrier or diluent that does not abrogate the biological activity and properties of the compound.

The pharmaceutical compositions described herein can be administered to a human patient *per se,* or in pharmaceutical compositions where they are mixed with other active ingredients, as in combination therapy, or suitable carriers or excipient(s). Techniques for formulation and administration of the compounds of the instant application may be found in "Remington's Pharmaceutical Sciences," Mack Publishing Co., Easton, PA, 18th edition, 1990.

Suitable routes of administration may, for example, include oral, rectal, transmucosal, or intestinal administration; parenteral delivery, including intramuscular, subcutaneous, intravenous, intramedullary injections, as well as intrathecal, direct intraventricular, intraperitoneal, intranasal, or intraocular injections.

Alternately, one may administer the compound in a local rather than systemic manner, for example, via injection of the compound directly in the renal or cardiac area, often in a depot or sustained release formulation. Furthermore, one may administer the drug in a targeted drug delivery system, for example, in a liposome coated with a tissue-specific antibody. The liposomes will be targeted to and taken up selectively by the organ.

The pharmaceutical compositions of the present invention may be manufactured in a manner that is itself known, e.g., by means of conventional mixing, dissolving, granulating, dragee-making, levigating, emulsifying, encapsulating, entrapping or tabeleting processes.

Pharmaceutical compositions for use in accordance with the present invention thus may be formulated in conventional manner using one or more physiologically acceptable carriers comprising excipients and auxiliaries which facilitate processing of the active compounds into preparations which can be used pharmaceutically. Proper formulation is dependent upon the route of administration chosen. Any of the well-known techniques, carriers, and excipients may be used as suitable and as understood in the art; e.g., in Remington's Pharmaceutical Sciences, above.

For injection, the agents of the invention may be formulated in aqueous solutions or lipid emulsions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained by mixing one or more solid excipient with pharmaceutical combination of the invention, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethyl cellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Furthermore, the formulations of the present invention may be coated with enteric polymers. All formulations for oral administration should be in dosages suitable for such administration.

For buccal administration, the compositions may take the form of tablets or lozenges formulated in conventional manner.

For administration by inhalation, the compounds for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, e.g., gelatin for use in an inhaler or insufflator may be formulated containing a powder mix of the compound and a suitable powder base such as lactose or starch.

The compounds may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active ingredient may be in powder form for constitution with a suitable vehicle, e.g., sterile pyrogen-free water, before use.

The compounds may also be formulated in rectal compositions such as suppositories or retention enemas, e.g., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

A pharmaceutical carrier for the hydrophobic compounds of the invention is a cosolvent system comprising benzyl alcohol, a nonpolar surfactant, a water-miscible organic polymer, and an aqueous phase. A common cosolvent system used is the VPD co-solvent system, which is a solution of 3% w/v benzyl alcohol, 8% w/v of the nonpolar surfactant Polysorbate 80™, and 65% w/v polyethylene glycol 300, made up to volume in absolute ethanol. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of POLYSORBATE 80™; the fraction size of polyethylene glycol may be varied; other biocompatible polymers may replace polyethylene glycol, e.g., polyvinyl pyrrolidone; and other sugars or polysaccharides may substitute for dextrose.

Alternatively, other delivery systems for hydrophobic pharmaceutical compounds may be employed. Liposomes and emulsions are well known examples of delivery vehicles or carriers for hydrophobic drugs. Certain organic solvents such as dimethylsulfoxide also may be employed, although usually at the cost of greater toxicity. Additionally, the compounds may be delivered using a sustained-release system, such as semipermeable matrices of solid hydrophobic polymers containing the therapeutic agent. Various sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

Some emulsions used in solubilizing and delivering the xanthine derivatives described above are discussed in U.S. Patent 6,210,687, which is incorporated by reference herein in its entirety, including any drawings.

Many of the compounds used in the pharmaceutical combinations of the invention may be provided as salts with pharmaceutically compatible counterions. Pharmaceutically compatible salts may be formed with many acids, including but not limited to hydrochloric, sulfuric, acetic, lactic, tartaric, malic, succinic, *etc.* Salts tend to be more soluble in aqueous or other protonic solvents than are the corresponding free acid or base forms.

Pharmaceutical compositions suitable for use in the present invention include compositions where the active ingredients are contained in an amount effective to achieve its intended purpose. More specifically, a therapeutically effective amount means an amount of compound effective to prevent, alleviate or ameliorate symptoms of disease or prolong the survival of the subject being treated. Determination of a therapeutically effective amount is well within the capability of those skilled in the art, especially in light of the detailed disclosure provided herein.

The exact formulation, route of administration and dosage for the pharmaceutical compositions of the present invention can be chosen by the individual physician in view of the patient's condition. (See *e.g.,* Fingl *et al.* 1975, in "The Pharmacological Basis of Therapeutics", Ch. 1 p. 1). Typically, the dose range of the composition administered to the patient can be from about 0.5 to 1000 mg/kg of the patient's body weight. The dosage may be a single one or a series of two or more given in the course of one or more days, as is needed by the patient.

The daily dosage regimen for an adult human patient may be, for example, an oral dose of between 0.1 mg and 500 mg, preferably between 1 mg and 250 mg, e.g. 5 to 200 mg or an intravenous, subcutaneous, or intramuscular dose of between 0.01 mg and 100 mg, preferably between 0.1 mg and 60 mg, e.g. 1 to 40 mg of the pharmaceutical compositions of the present invention or a pharmaceutically acceptable salt thereof calculated as the free base, the composition being administered 1 to 4 times per day. Alternatively the compositions of the invention may be administered by continuous intravenous infusion, preferably at a dose of up to 400 mg per day. Thus, the total daily dosage by oral administration will be in the range 1 to 2000 mg and the total daily dosage by parenteral administration will be in the range 0.1 to 400 mg. Suitably the compounds will be administered for a period of continuous therapy, for example for a week or more, or for months or years.

Dosage amount and interval may be adjusted individually to provide plasma levels of the active moiety which are sufficient to maintain the modulating effects, or minimal effective concentration (MEC). The MEC will vary for each compound but can be estimated from in vitro data. Dosages necessary to achieve the MEC will depend on individual characteristics and route of administration. However, HPLC assays or bioassays can be used to determine plasma concentrations.

Dosage intervals can also be determined using MEC value. Compositions should be administered using a regimen which maintains plasma levels above the MEC for 10-90% of the time, preferably between 30-90% and most preferably between 50-90%.

In cases of local administration or selective uptake, the effective local concentration of the drug may not be related to plasma concentration.

The amount of composition administered will, of course, be dependent on the subject being treated, on the subject's weight, the severity of the affliction, the manner of administration and the judgment of the prescribing physician.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Compositions comprising a compound of the invention formulated in a compatible pharmaceutical carrier may also be prepared, placed in an appropriate container, and labeled for treatment of an indicated condition.

## Claims

1. Adenosine A₁ receptor antagonist (AA₁RA) for use in treating alkalosis in a patient.

2. AA₁RA as defined in claim 1, wherein said alkalosis is metabolic alkalosis or respiratory alkalosis.

3. AA₁RA as defined in claim 1 or 2, wherein said patient has edema, is on diuretic therapy, or suffers from acid loss through the patient's upper gastrointestinal tract.

4. AA₁RA as defined in any previous claim which is a xanthine-derivative compound of Formula I or a pharmaceutically acceptable salt thereof, 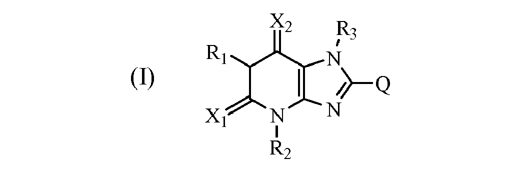 wherein
each of X₁ and X₂ independently represents oxygen or sulfur;
Q represents: 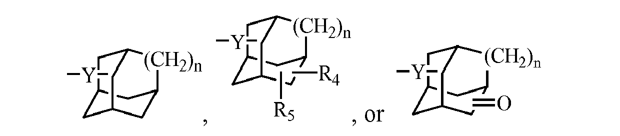 where Y represents a single bond or alkylene having 1 to 4 carbon atoms, n represents 0 or 1;
each of R₁ and R₂ independently represents hydrogen, lower alkyl, allyl, propargyl, or hydroxy-substituted, oxo-substituted or unsubstituted lower alkyl, and R₃ represents hydrogen or lower alkyl, or
R₄ and R₅ are the same or different and each represent hydrogen or hydroxy, and when both R₄ and R₅ are hydrogen, at least one of R₁ and R₂ is hydroxy-substituted or oxo-substituted lower alkyl, provided that when Q is 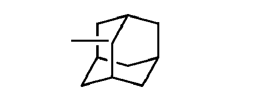 then R₁, R₂ and R₃ are not simultaneously methyl,
or wherein said AA₁RA is a xanthine epoxide-derivative compound of Formula II or Formula III, or a pharmaceutically acceptable salt thereof, 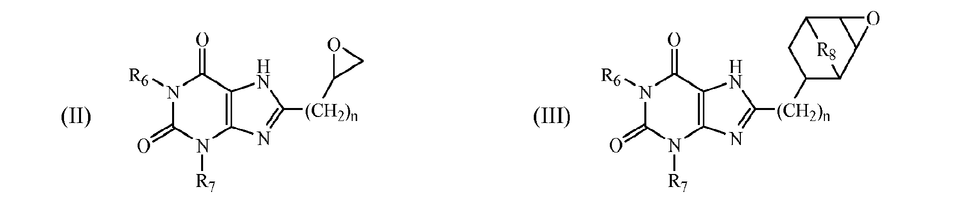 wherein R₆ and R₇ are the same or different, and can be hydrogen or an alkyl group of 1-4 carbons, R₈ is either oxygen or (CH₂)₁₋₄, and n=0-4.

5. AA₁RA as defined in any previous claim which is selected from the group consisting of 8-(noradamantan-3-yl)-1,3-dipropylxanthine; 1,3-Diallyl-8-(3-noradamantyl)xanthine, 3-allyl-8-(3-noradamantyl)-1-propargylxanthine, 8-(trans-9-hydroxy-3-tricyclo[3.3.1.0^{3,7}]nonyl) -1,3-dipropylxanthine, 8-(cis-9-hydroxy-3-tricyclo[3.3.1.0^{3,7}]nonyl)-1,3-dipropylxanthine, 8-(trans-9-hydroxy-3-tricyclo[3.3.1.0^{3,7}]nonyl)-1-(2-oxopropyl)-3-propylxanthine, 1-(2-hydroxypropyl)-8-(trans-9-hydroxy-3-tricyclo[3.3.1.0^{3,7}]nonyl)-3-propylxanthine, 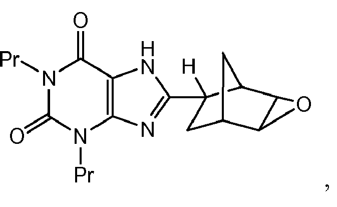 and 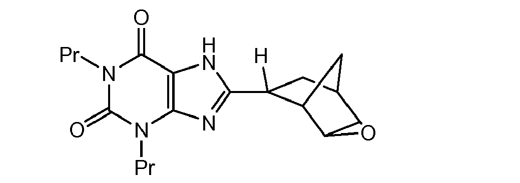 or a pharmaceutically acceptable salt thereof.

6. The use of a AA₁RA as defined in any previous claim for the manufacture of a medicament for treating alkalosis in a patient.

7. The use of claim 6 wherein said alkalosis is metabolic alkalosis or respiratory alkalosis.

8. The use of claim 6 or 7, wherein said patient has edema, is on diuretic therapy, or suffers from acid loss through the patient's upper gastrointestinal tract.
